# EUROPEAN PATENT APPLICATION

(11) **EP 1 691 537 A1**
(43) Date of publication of application: **16.08.2006**
(21) Application number: 06002965.9
(22) Date of filing: 14.02.2006
(51) Int. Cl.: H04M 1/725, G10H 1/00, H04M 1/247

(54) **Apparatus and method for performing a music play function in a portable terminal**

(30) Priority: 14.02.2005 KR 2005012051
(71) Applicant: Samsung Electronics Co., Ltd., Yeongtong-gu Suwon-si, Gyeonggi-do (KR)
(72) Inventor: Kim, Ki-Uk c/o Samsung Electronics Co., Ltd.,, Suwon-si, Gyeonggi-do (KR)
(74) Representative: Grünecker, Kinkeldey, Stockmair & Schwanhäusser Anwaltssozietät

(57) **Abstract**

An apparatus and method is provided for performing a play function in a portable terminal. The apparatus and method includes a memory to store a sound combination table in which preset play sounds and their associated key input combination patterns are stored, to reproduce a particular play sound. A keypad is included to receive a plurality of key inputs for reproducing the play sound, and an audio processor is provided to reproduce play sounds corresponding to the plurality of key inputs. A controller is provided to then analyze the plurality of key input combination patterns, extract play sounds mapped to the key input combination patterns using the sound combination table, and reproduce the extracted play sounds.

## Description

### BACKGROUND OF THE INVENTION

### Field of the Invention:

The present invention relates generally to a portable terminal. In particular, the present invention relates to an apparatus and method for supporting a play function (or music playing function) in a portable terminal.

### Description of the Related Art:

Compared with analog wireless communication, typically called the "1^{st} generation (1G) wireless communication," digital communication, typically called the "2^{nd} generation (2G) wireless communication", transmits low-speed data as well as voice, with a wireless network-centered service. This wireless network-centered 2G mobile communication service has been evolving into a 4^{th} generation (4G) mobile communication service, distinguished by its super high speed, high capacity and mobility, via the 3^{rd} generation (3G) mobile communication service (also known as International Mobile Telecommunication-2000 (IMT-2000)), that is capable of supporting wire/wireless integrated high-speed Internet and multimedia services. The advent of the various new mobile communication services has contributed to the popularization of various additional services that every one can enjoy regardless of time and place. In addition, the portable terminal technology has steadily developed along with the evolution of the mobile communication service. The portable terminal for the information society based on digitalization and networking has been evolving so as to enable real-time services for data and image, as well as voice, thereby developing into a multi-function, high-speed, small-sized terminal for easy carrying.

The typical portable terminal includes devices such as a cellular phone, Personal Digital Assistant (PDA), Hand Held Phone (HHP), digital communication device, portable game terminal, and so forth. A service subscriber carrying the portable terminal can exchange desired information and receive various data services regardless of time and place. To this end, the portable terminal shows a tendency to include features such as miniaturization, slimness, easy grip and light weight, and tends to be easily modified so as to adapt to various multimedia environments and an Internet environment due to the evolution of the mobile communication technology.

Therefore, the portable terminal has become a necessity of modem living, and a need has developed for portable terminals including various functions such as data communication, game features, and motion image reproduction. The various desired functions may further include a function for reproducing sounds using a predetermined sound source. For example, early portable terminals supported only single-tone bell or ring sounds, but with the development of a sound chip, up-to-date portable terminals can often support polyphonic bell or ring sounds of 40 or more poly sounds. Along with the development of the sound chip, portable terminals with built-in MP3 players and portable terminals with sound reproduction functions have been introduced. Although some recent portable terminals have a function for supporting various sounds, there has been substantially no portable terminal developed to provide a play function of musical instruments.

Accordingly, a need exists for a portable terminal system and method for providing a play function of musical instruments. In addition, because the current user interface cannot fully express various sounds, there is a need for a portable terminal system and method that is capable of playing music with various tones.

### SUMMARY OF THE INVENTION

It is, therefore, an object of embodiments of the present invention to substantially solve the above and other problems, and provide an apparatus and method for performing a play function in a portable terminal.

It is another object of embodiments of the present invention to provide an apparatus and method for performing a play function by reproducing various sounds through a limited user interface in a portable terminal.

According to one aspect of embodiments of the present invention, an apparatus is provided for performing a play function in a portable terminal. The apparatus comprises a memory for storing a sound combination table in which preset play sounds and their associated key input combination patterns are stored, to reproduce a particular play sound, a keypad for receiving a plurality of key inputs for reproducing the play sound, an audio processor for reproducing play sounds corresponding to the plurality of key inputs, and a controller for analyzing the plurality of key input combination patterns, extracting play sounds mapped to the key input combination patterns using the sound combination table, and reproducing the extracted play sounds.

According to one aspect of embodiments of the present invention, a method is provided for performing a play function in a portable terminal. The method comprises the steps of setting a play mode and receiving a plurality of key inputs from the exterior, analyzing a plurality of key input combination patterns mapped to the plurality of key inputs and extracting particular play sound from the key input combination patterns using a sound combination table in which preset play sounds and their associated key input combination patterns are stored, and reproducing the extracted play sound.

### BRIEF DESCRIPTION OF THE DRAWINGS

The above and other objects, features and advantages of embodiments of the present invention will become more apparent from the following detailed description when taken in conjunction with the accompanying drawings, in which:
FIG. 1 is a block diagram illustrating a structure of an exemplary portable terminal with a play function according to an embodiment of the present invention;
FIG. 2A is a perspective view illustrating an external appearance of an exemplary portable terminal with the folder opened, having a play function according to an embodiment of the present invention;
FIG. 2B is a perspective view illustrating an external appearance of the portable terminal of FIG. 2A with the folder closed, having a play function according to an embodiment of the present invention;
FIG. 3 is a view illustrating an exemplary display screen of a portable terminal with a play function according to an embodiment of the present invention;
FIG. 4 is a flowchart illustrating an exemplary operation of a portable terminal with a play function according to an embodiment of the present invention; and
FIG. 5 is a view illustrating an application operation of a portable terminal with a play function according to an embodiment of the present invention.

Throughout the drawings, like reference numerals will be understood to refer to like parts, components and structures.

### DETAILED DESCRIPTION OF EXEMPLARY EMBODIMENTS

Exemplary embodiments of the present invention will now be described in detail with reference to the annexed drawings. In the following description, a detailed description of known functions and configurations incorporated herein has been omitted for clarity and conciseness.

Embodiments of the present invention comprise an apparatus and method for providing a play function that is capable of reproducing play sounds in response to key inputs in a portable terminal. In particular, embodiments of the present invention comprise an apparatus and method for generating key input combination patterns in response to key inputs, extracting play sounds mapped to the key input combination patterns, and reproducing the extracted play sounds.

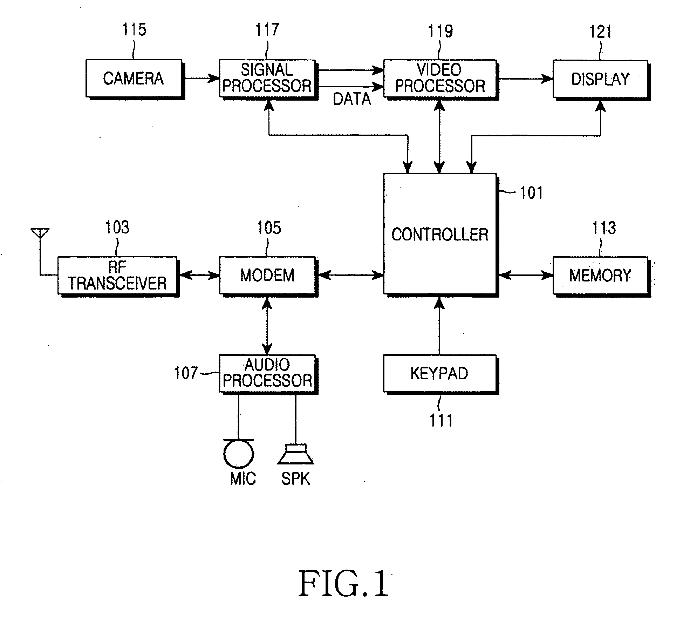

FIG. 1 is a block diagram illustrating an exemplary structure of a portable terminal with a play function according to an embodiment of the present invention.

Referring to FIG. 1, a portable terminal comprises a controller 101, a radio frequency (RF) transceiver 103, a modem 105, an audio processor 107, a keypad 111, a memory 113, a camera 115, a signal processor 117, a video processor 119 and a display 121. The portable terminal can further comprise a microphone (MIC) and a speaker (SPK). The radio frequency (RF) transceiver 103 performs a radio communication function of the portable terminal. The RF transceiver 103 can comprise an RF transmitter for up-converting a frequency of a transmission signal and amplifying the up-converted transmission signal, and an RF receiver for low-noise-amplifying a received signal and down-converting a frequency of the low-noise-amplified signal.

The modem 105 can comprise a transmitter for encoding and modulating the transmission signal, and a receiver for demodulating and decoding the received signal.

The audio processor 107 can be implemented using a codec for example, and the codec can comprise a data codec for processing packet data and an audio codec for processing an audio signal such as voices. The audio processor 107 converts a digital audio signal received from the modem 105 into an analog audio signal through the audio codec, and reproduces the analog audio signal. In addition, the audio processor 107 converts an analog audio signal generated from the microphone (MIC) into a digital audio signal through the audio codec, and transmits the digital audio signal to the modem 105. The codec can be separately provided or included in the controller 101.

The keypad 111 can comprise alphanumeric keys used for inputting numeral and text information and function keys used for setting various functions. The keypad 111 may further comprise play keys used for performing the play function according to an embodiment of the present invention. The play keys can be set on the existing keypad, or separate play keys can be added.

The memory 113 can comprise a program memory and a data memory. The program memory stores programs for controlling the general operation of the portable terminal, and the data memory temporarily stores the data generated during the execution of the programs.

The controller 101 performs a function for controlling the overall operation of the portable terminal. As described above, the controller 101 can comprise the modem 105 and the codec. In addition, the controller 101 controls a process for performing the play function according to an embodiment of the present invention.

The camera 115 can comprise a camera sensor for photographing an image and converting the photographed optical signal into an electric signal. Herein, the camera sensor can be implemented using a charge coupled device (CCD) sensor for example.

The signal processor 117 converts a video signal output from the camera 115 into an image signal. The signal processor 117 can be implemented using a digital signal processor (DSP) for example.

The video processor 119 performs a function for generating screen data for displaying the video signal output from the signal processor 117. The video processor 119, under the control of the controller 101, transmits received video signal and data according to the specifications of the display 121.

The display 121 displays the video data output from the video processor 119. Herein, the display 121 can be implemented using a liquid crystal display (LCD) for example. In this case, the display 121 can comprise an LCD controller, a memory for storing the video data, and an LCD device. When the LCD is implemented on a touch screen basis, the keypad 111 and the LCD can serve as an input unit.

Embodiments of the present invention apply a play function to the portable terminal having the structure described above. With reference to FIG. 1, a description will now be made of a play function of the portable terminal according to an exemplary embodiment of the present invention.

A portable terminal according to an embodiment of the present invention receives key inputs from a user through the keypad 111 in order to reproduce play sounds for performing the play function. The keypad 111 can comprise play keys used for reproducing play sounds, and play setting keys used for setting half tones or octaves for the play sounds. As described above, the play keys and the play setting keys can be implemented using the existing keys provided in the portable terminal, or the play keys and the play setting keys can be separately provided to perform the play function.

If there are key inputs from the keypad 111, the controller 101 analyzes key input combination patterns for the key inputs, loads a sound combination table stored in the memory 113, and extracts play sounds mapped to the key input combination patterns. The sound combination table is generated by mapping preset play sounds to their associated key input combination patterns to generate particular sounds, and are then stored in the memory 113.

Table 1 below shows, by way of example, an exemplary sound combination table generated using three input buttons of the keypad 111 to represent play sounds.

**Table 1**

| Key Input Combination Patterns (Play Keys) | Play Sounds |
|---|---|
| P O O | Do |
| O P O | Re |
| O O P | Mi |
| O P P | Fa |
| P P O | Sol |
| P O P | La |
| P P P | Si |

Referring to Table 1, the three input buttons of the keypad 111 are mapped to each of the play sounds. In accordance with an exemplary embodiment of the present invention, the play sounds comprise the syllables or pitches assigned to degrees or steps of the diatonic scale used in solfege of solmization. Herein, 'P' denotes an operating key and 'O' denotes a non-operating key. For example, if only the center key among the play keys operates, the play sound becomes a 'Re' sound.

The portable terminal also receives the play setting key inputs, along with the key input combination patterns of the play keys. The controller 101 sets octaves or half tones to the play setting keys to extend the 7 play sounds of Table 1, thereby reproducing wideband sounds. That is, if there is a play setting key input for octave setting, the controller 101 sets an octave according to the corresponding key input. However, if there is no play setting key input for octave setting, the controller 101 keeps the current octave. Similarly, if there is a play setting key input for half-tone setting, the controller 101 sets a half tone for the play sound generated by the play keys. In addition to the method of setting octaves and half tones, embodiments of the present invention can apply other function keys.

In this operation, the controller 101 extracts play sounds to be reproduced, and transmits the extracted play sounds to the modem 105. The modem 105 encodes the input signal (that is, the play sounds), and transmits the encoded signal to the audio processor 107. The audio processor 107 converts the digital audio signal (that is, the play sounds) received from the modem 105 into an analog signal through the audio codec, and reproduces the analog signal. In reproducing the play sounds, the controller 101 sets half tones or octaves according to the play setting key inputs, and applies tones of various instruments when an instrument function is applied.

Alternatively, embodiments of the present invention can apply a function for previously storing the play sounds being played in the memory 113, and later playing back the play sounds stored in the memory 113. In addition, the controller 101 performs a control operation for displaying on the display 121, information on scale, octave and half tone of the play sounds being reproduced through the audio processor 107, and the display 121 displays the play sound information under the control of the controller 101.

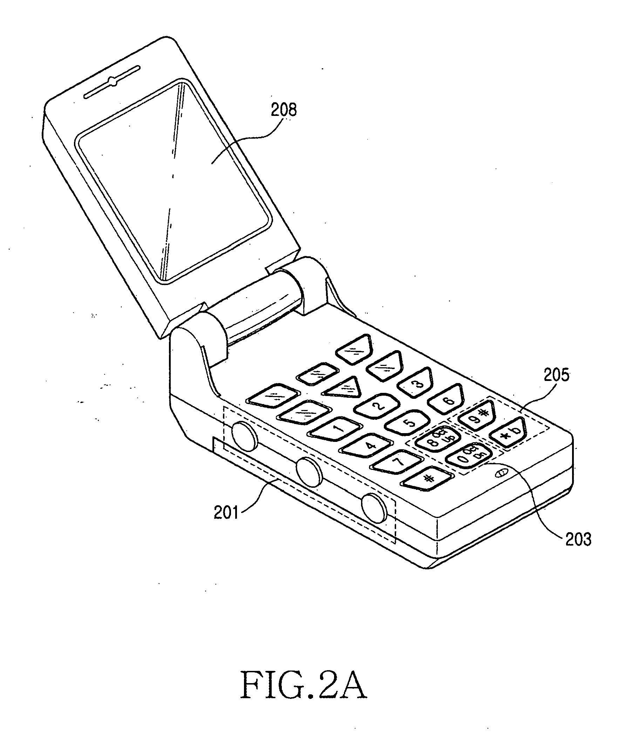

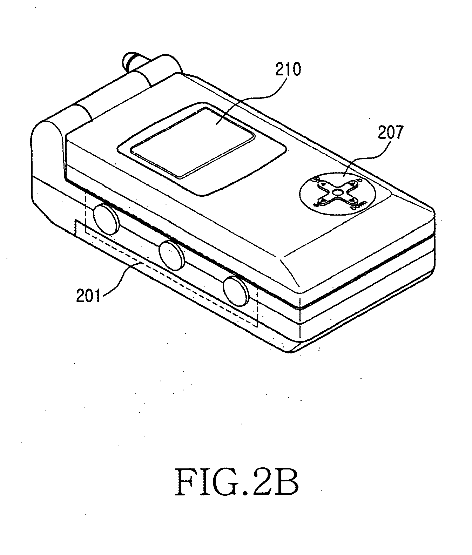

With reference to FIGs. 2A and 2B, a description will now be made of an exemplary keypad to which the play keys used for reproducing play sounds and the play setting keys are applied according to an embodiment of the present invention.

FIG. 2A is a perspective view illustrating an external appearance of an exemplary portable terminal with the folder opened, having a play function according to an embodiment of the present invention. FIG. 2B is a perspective view illustrating an external appearance of the portable terminal of FIG. 2A with the folder closed, having a play function according to an embodiment of the present invention.

Referring to FIGs. 2A and 2B, the portable terminal comprises play keys 201 used for reproducing play sounds, and which are mounted on one side thereof. Although the embodiment of the present invention illustrated in FIGs. 2A and 2B uses three play keys, embodiments of the present invention are not limited thereto and the number, location, and arrangement of the play keys is subject to change. The sound combination table can be freely modified depending on the number of the play keys 201. In addition, although the play keys 201 are mounted on one side of the portable terminal for playing convenience, the play keys 201 can also be set on the existing keypad through user setting. The portable terminal of FIGs. 2A and 2B further comprises a main display 208, shown in the folder-opened state of the device of Fig. 2A, and a subordinate display 210, shown in the folder-closed state of the device of FIG. 2B.

The portable terminal according to an embodiment of the present invention comprises play setting keys 203, 205 and 207. In the device state illustrated in FIG. 2A in which the folder is opened, the user can set an octave using the play setting keys 203 for setting the octave up or down, and can set half tones for the play sounds using the play setting keys 205 for setting half tones. In the device state illustrated in FIG. 2B in which the folder is closed, the user can set octaves and half tones using the 4-way play setting key 207 separately mounted on an outer surface of the folder. Further, in the device state illustrated in FIG. 2A in which the folder is opened, the user can also set octaves and half tones using the 4-way play setting key 207 separately mounted on the outer surface of the folder should the user desire to do so.

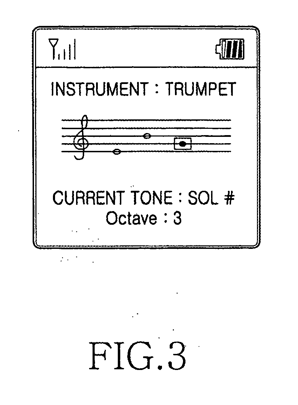

FIG. 3 is a diagram schematically illustrating an exemplary display screen of a portable terminal with a play function according to an embodiment of the present invention.

Referring to FIG. 3, the screen displays information indicating that the portable terminal is reproducing a 'Sol' sound, and has set a half tone '#', set a 3^{rd} octave, and is using a trumpet as an instrument. In this way, embodiments of the present invention can display the currently-played sound information on the main display 208 (for example, in the folder-opened state of the device of Fig. 2A) or the subordinate display 210 (for example, in the folder-closed state of the device of FIG. 2B) of the portable terminal.

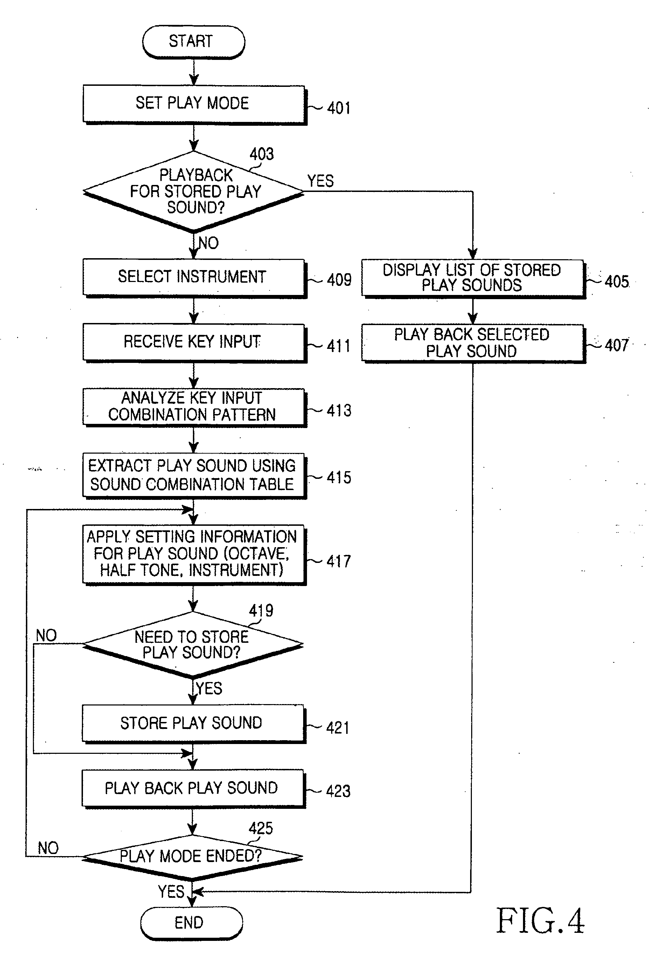

FIG. 4 is a flowchart illustrating an exemplary operation of a portable terminal with a play function according to an embodiment of the present invention.

Referring to FIG. 4, a portable terminal sets a play mode to reproduce play sounds in step 401. The portable terminal determines in step 403 whether a user desires to play back the play sounds previously played and stored by the user in the play mode. If it is determined that the user desires to play back the stored play sounds, the portable terminal displays in step 405, a list of the stored play sounds and allows the user to select desired play sounds from the play sound list using a hot key. The portable terminal then plays back the selected play sounds in step 407.

However, if it is determined that the user does not desire to play back the stored play sounds, the portable terminal allows the user to reproduce play sounds using various instruments. To this end, the portable terminal displays in step 409, a list of available instruments and allows the user to select an instrument from the instrument list to be used in reproducing the play sounds. If the selected instrument is a trumpet for example, the portable terminal allows the user to reproduce the play sounds with trumpet tones.

In step 411, the portable terminal receives key inputs for the play keys or the play setting keys from the user through the keypad. In step 413, the portable terminal analyzes key input combination patterns of the received play key inputs, and also analyzes the received play setting key inputs. After the analysis of the key input combination patterns, the portable terminal extracts play sounds using a sound combination table in step 415, and in step 417 applies setting information for the extracted play sounds, such as octave and half tone, and instrument information.

The portable terminal then determines in step 419 whether to store the play sounds. If it is determined to store the play sounds, the portable terminal stores the play sounds in step 421. The stored play sounds can be used as bell or ring sounds for example, or played back when necessary. Thereafter, the portable terminal plays back the play sounds in step 423. The portable terminal determines in step 425 whether the play mode is ended. If it is determined that the play mode is ended, the portable terminal ends the operation. However, if the play mode is not ended, the portable terminal returns to step 417 to receive key inputs for the next play sounds. In this manner, the portable terminal according to an embodiment of the present invention, can reproduce play sounds according to the key inputs, and can also set octaves and half tones to reproduce various sounds.

The portable terminal with a play function provides various application operations for the play function. In addition, the user can play various games using the portable terminal with a play function.

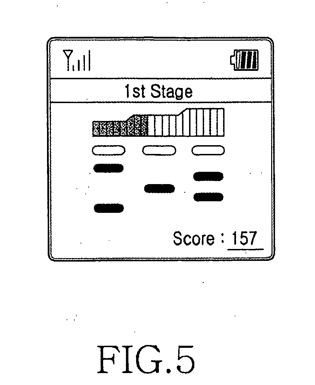

FIG. 5 is a view illustrating an application operation of a portable terminal with a play function according to an embodiment of the present invention.

Referring to FIG. 5, if the portable terminal displays key input information indicating key manipulation for each key with a time interval, the user can play a game 'Pump' of playing music through key inputting for example.

In addition to the exemplary game shown in FIG. 5, the user can use the portable terminal with a play function for other various applications.

Although embodiments of the present invention have been described with reference to a folder-type portable terminal with three play keys and play setting keys for setting octaves and half tones, it can also be applied to different types of portable terminals, for example, terminals that use the existing keypad or a separate keypad for the play function. In addition, it is also possible to increase the number of play keys and accordingly, modify the sound combination table.

As can be understood from the foregoing description, the exemplary embodiments of the portable terminal with a play function receive key inputs through a keypad and reproduce play sounds using the received key inputs based on the sound combination table. Therefore, the portable terminal with a play function can reproduce various play sounds using its limited user interface. In addition, several users can play in concert using their own portable terminals with the play function.

While the present invention has been shown and described with reference to certain exemplary embodiments thereof, it will be understood by those skilled in the art that various changes in form and detail may be made therein without departing from the spirit and scope of the invention as defined by the appended claims.

## Claims

1. An apparatus for performing a play function in a portable terminal, the apparatus comprising:
a memory for storing a sound combination table in which preset play sounds and their associated key input combination patterns are stored;
a keypad for receiving a plurality of key inputs for reproducing the play sound;
an audio processor for reproducing play sounds corresponding to the plurality of key inputs; and
a controller for analyzing the plurality of key input combination patterns, extracting play sounds mapped to the key input combination patterns using the sound combination table, and reproducing the extracted play sounds.

2. The apparatus of claim 1, wherein the keypad comprises:
play keys for receiving the key sounds; and
play setting keys for setting octaves and half tones for the play sounds.

3. The apparatus of claim 1, further comprising a display for displaying information on the play sounds being reproduced.

4. The apparatus of claim 1, wherein the memory is configured to store therein the play sounds reproduced by the audio processor.

5. The apparatus of claim 1, wherein the play sounds are reproduced with tones of any selected one of a predetermined number of instruments.

6. The apparatus of claim 1, wherein the play sounds are reproduced with tones of any of a predetermined number of instruments.

7. The apparatus of claim 1, wherein the play sounds correspond to respective ones of syllables or pitches assigned to steps in a diatonic scale.

8. The apparatus of claim 1, wherein octaves and half tones are applied to the play sounds being reproduced.

9. A method for performing a play function in a portable terminal, the method comprising the steps of:
setting a play mode and receiving a plurality of key inputs;
analyzing a plurality of key input combination patterns mapped to the plurality of key inputs, and extracting a particular play sound from the key input combination patterns using a sound combination table in which preset play sounds and their associated key input combination patterns are stored; and
reproducing the extracted play sound.

10. The method of claim 9, further comprising the step of:
if there is any key input for setting octaves or half tones for the play sound reproduction, setting octaves or half tones according to the key input and reproducing the play sound.

11. The method of claim 9, further comprising the step of displaying information on the play sounds being reproduced.

12. The method of claim 9, further comprising the steps of:
determining whether to store the play sounds being displayed; and
storing the play sounds according to the determination result.

13. The method of claim 12, further comprising the steps of:
determining whether to play back the play sounds stored in the play mode; and
playing back the stored play sounds according to the determination result.

14. The method of claim 9, wherein the play sound reproducing step comprises the steps of:
selecting an instrument to be used for the play sounds; and
reproducing the play sounds with tones of the selected instrument.

15. The method of claim 9, wherein the play sounds correspond to respective ones of syllables or pitches assigned to steps in a diatonic scale.

16. The method of claim 9, wherein the play sound reproducing step comprises the steps of:
selecting at least one instrument to be used for the play sounds; and
reproducing the play sounds with tones of the selected at least one instrument.

17. A computer program embodied on a computer-readable medium for performing a play function in a portable terminal, comprising:
a first set of instructions for setting a play mode and receiving a plurality of key inputs;
a second set of instructions for analyzing a plurality of key input combination patterns mapped to the plurality of key inputs, and extracting a particular play sound from the key input combination patterns using a sound combination table in which preset play sounds and their associated key input combination patterns are stored; and
a third set of instructions for reproducing the extracted play sound.

18. The computer program embodied on a computer-readable medium of claim 17, further comprising:
a set of instruction for setting octaves or half tones according to the key input and reproducing the play sound if there is any key input for setting octaves or half tones for the play sound reproduction; and
a set of instructions for displaying information on the play sounds being reproduced.

19. The computer program embodied on a computer-readable medium of claim 17, further comprising:
a set of instruction for determining whether to store the play sounds being displayed and storing the play sounds according to the determination result; and
a set of instructions for determining whether to play back the play sounds stored in the play mode and playing back the stored play sounds according to the determination result.

20. The computer program embodied on a computer-readable medium of claim 17, further comprising:
a set of instruction for selecting at least one instrument to be used for the play sounds and reproducing the play sounds with tones of the selected at least one instrument.
